# EUROPEAN PATENT SPECIFICATION

(11) **EP 3 082 615 B1**
(45) Date of publication and mention of the grant of the patent: **27.11.2019**
(21) Application number: 14821848.0
(22) Date of filing: 28.11.2014
(51) Int. Cl.: A61B 8/08, G06T 7/00, G06K 9/62, G06T 7/60

(54) **AUTOMATIC ULTRASOUND BEAM STEERING AND NEEDLE ARTIFACT SUPPRESSION**
AUTOMATISCHE ULTRASCHALLSTRAHLSTEUERUNG UND NADELARTEFAKTUNTERDRÜCKUNG
ORIENTATION AUTOMATIQUE DE FAISCEAU D'ULTRASONS ET SUPPRESSION D'ARTEFACTS D'AIGUILLE

(30) Priority: 20.12.2013 US 201361918912 P; 30.06.2014 US 201462019087 P
(43) Date of publication of application: 26.10.2016
(73) Proprietor: Koninklijke Philips N.V., 5656 AE Eindhoven (NL)
(72) Inventor: PARTHASARATHY, Vijay, NL-5656 AE Eindhoven (NL); NG, Gary Cheng-How, NL-5656 AE Eindhoven (NL); HATT, Charles Ray, NL-5656 AE Eindhoven (NL)
(74) Representative: Philips Intellectual Property & Standards
(86) International application number: PCT/IB2014/066411
(87) International publication number: WO 2015/092582

(56) References cited:
- COOL DEREK ET AL: "Temporal-based needle segmentation algorithm for transrectal ultrasound prostate biopsy procedures", MEDICAL PHYSICS, AIP, MELVILLE, NY, US, vol. 37, no. 4, 18 March 2010 (2010-03-18) , pages 1660-1673, XP012135694, ISSN: 0094-2405, DOI: 10.1118/1.3360440
- DING MINGYUE ET AL: "Automatic needle segmentation in three-dimensional ultrasound images using two orthogonal two-dimensional image projections", MEDICAL PHYSICS, AIP, MELVILLE, NY, US, vol. 30, no. 2, 1 February 2003 (2003-02-01), pages 222-234, XP012011986, ISSN: 0094-2405, DOI: 10.1118/1.1538231
- ALPER AYVACI ET AL: "Biopsy needle detection in transrectal ultrasound", COMPUTERIZED MEDICAL IMAGING AND GRAPHICS, PERGAMON PRESS, NEW YORK, NY, US, vol. 35, no. 7, 24 March 2011 (2011-03-24) , pages 653-659, XP028282344, ISSN: 0895-6111, DOI: 10.1016/J.COMPMEDIMAG.2011.03.005 [retrieved on 2011-04-08]
- OKAZAWA S H ET AL: "Methods for segmenting curved needles in ultrasound images", MEDICAL IMAGE ANALYSIS, OXFORD UNIVERSITY PRESS, OXOFRD, GB, vol. 10, no. 3, 1 June 2006 (2006-06-01), pages 330-342, XP028013459, ISSN: 1361-8415, DOI: 10.1016/J.MEDIA.2006.01.002 [retrieved on 2006-06-01]
- WU QIU ET AL: "Needle Segmentation Using 3D Quick Randomized Hough Transform", INTELLIGENT NETWORKS AND INTELLIGENT SYSTEMS, 2008. ICINIS '08. FIRST INTERNATIONAL WORKSHOP ON, IEEE, PISCATAWAY, NJ, USA, 1 November 2008 (2008-11-01), pages 449-452, XP031364313, ISBN: 978-0-7695-3391-9
- CARNEIRO G ET AL: "Detection and Measurement of Fetal Anatomies from Ultrasound Images using a Constrained Probabilistic Boosting Tree", IEEE TRANSACTIONS ON MEDICAL IMAGING, IEEE SERVICE CENTER, PISCATAWAY, NJ, US, vol. 27, no. 9, 1 September 2008 (2008-09-01), pages 1342-1355, XP011232034, ISSN: 0278-0062, DOI: 10.1109/TMI.2008.928917
- CARNEIRO G ET AL: "The Segmentation of the Left Ventricle of the Heart From Ultrasound Data Using Deep Learning Architectures and Derivative-Based Search Methods", IEEE TRANSACTIONS ON IMAGE PROCESSING, IEEE SERVICE CENTER, PISCATAWAY, NJ, US, vol. 21, no. 3, 1 March 2012 (2012-03-01), pages 968-982, XP011491945, ISSN: 1057-7149, DOI: 10.1109/TIP.2011.2169273
- CHARLES R. HATT ET AL: "Enhanced needle localization in ultrasound using beam steering and learning-based segmentation", COMPUTERIZED MEDICAL IMAGING AND GRAPHICS, vol. 41, 6 July 2014 (2014-07-06), pages 46-54, XP055175101, ISSN: 0895-6111, DOI: 10.1016/j.compmedimag.2014.06.016

## Description

### FIELD OF THE INVENTION

The present invention relates to segmenting a medical instrument in an ultrasound image and, more particularly, to dynamically performing the segmentation responsive to acquiring the image. Performing "dynamically" or in "real time" is interpreted in this patent application as completing the data processing task without intentional delay, given the processing limitations of the system and the time required to accurately measure the data needed for completing the task.

### BACKGROUND OF THE INVENTION

Ultrasound (US) image guidance increases the safety and efficiency of needle guided procedures by enabling real-time visualization of needle position within the anatomical context. The ability to use ultrasound methods like electronic beam steering to enhance the visibility of the needle in ultrasound-guided procedures has become a significant competitive area in the past few years.

While real-time 3D ultrasound is available, 2DUS is much more widely used for needle-based clinical procedures due to its increased availability and simplified visualization capabilities.

With 2DUS, it is possible to electronically steer the US beam in a lateral direction perpendicular to the needle orientation, producing strong specular reflections that enhance needle visualization dramatically.

Since the current orientation of the probe with respect to the needle is not typically known at the outset, the beam steering angle needed to achieve normality with the needle is also unknown.

Also, visualization is difficult when the needle is not directly aligned within the US imaging plane and/or the background tissue contains other linear specular reflectors such as bone, fascia, or tissue boundaries.

In addition, artifacts in the image come about for various reasons, e.g., grating lobes from steering a linear array at large angles and specular echoes from the above-mentioned linear and other specular reflectors offering a sharp attenuation change to ultrasound incident at, or close to, 90 degrees.

"Enhancement of Needle Visibility in Ultrasound-Guided Percutaneous Procedures" by Cheung et al. (hereinafter "Cheung") discloses automatic segmenting of the needle in an ultrasound image and determining the optimum beam steering angle.

Problematically, specular structures that resemble a needle interfere with needle detection. Speckle noise and imaging artifacts can also hamper the detection.

The solution in Cheung is for the user to jiggle the needle, thereby aiding the segmentation based on difference images.

In addition, Cheung requires user interaction in switching among modes that differ as to the scope of search for the needle. For example, a user reset of the search scope is needed when the needle is lost from view.

Cheung segmentation also relies on intensity-based edge detection that employs a threshold having a narrow range of effectiveness.

In "Temporal-based needle segmentation algorithm for transrectal ultrasound prostate biopsy procedures" by Derek W. Cool et al., Medical Physics, AIP, Melville, NY, US, vol. 37, no. 4, pp. 1660-1673, 18 March 2010, XP012135694, it is disclosed a temporal-based transrectal ultrasound (TRUS) needle segmentation algorithm for accurate segmentation of the biopsy needle and needle tip from TRUS biopsy images. The algorithm automatically identifies a TRUS image containing the biopsy needle from a collection of 2D TRUS images and segments the biopsy-core location from the 2D TRUS image.

On the other hand, "Detection and Measurement of Fetal Anatomies from Ultrasound Images using a Constrained Probabilistic Boosting Tree" by G. Carneiro et al., IEEE Transactions on Medical Imaging, Service Center, Piscataway, NJ, US, vol. 27, no. 9, pp. 1342-1355, 1 September 2008, XP011232034, describes a method for fast automatic detection and measurement of fetal anatomical structures in ultrasound images that directly exploits a large database of expert annotated fetal anatomical structures in ultrasound images. The method learns automatically to distinguish between the appearance of the object of interest and background by training a constrained probabilistic boosting tree classifier.

### SUMMARY OF THE INVENTION

What is proposed herein below addresses one or more of the above concerns.

In addition to above-noted visualization difficulties, visualization is problematic when the needle is not yet deeply inserted into the tissue. The Cheung difficulty in distinguishing the needle from "needle-like" specular deflector is exacerbated in detecting a small portion of the needle, as when the needle insertion is just entering the field of view. In particular, Cheung applies a Hough transform to edge detection output of the ultrasound image. Specular structures competing with the needle portion may appear longer, especially at the onset of needle entry into the field of view. They may therefore accumulate more votes in the Hough transform, and thereby be identified as the most prominent straight-line feature in the ultrasound image, i.e., the needle.

Yet, the clinical value of needle detection is questionable if, to determine the needle's pose, there is a need to wait until the needle is more deeply inserted. It would be better if the needle could be detected earlier in the insertion process, when the physician can evaluate its trajectory and change course without causing more damage and pain.

Reliable needle segmentation would allow automatic setting of the optimal beam steering angle, time gain compensation, and the image processing parameters, resulting in potentially enhanced visualization and clinical workflow.

In addition, segmentation and detection of the needle may allow fusion of ultrasound images with pre-operative modalities such as computed tomography (CT) or magnetic resonance (MR) imaging, enabling specialized image fusion systems for needle-based procedures.

A technological solution is needed to automatic needle segmentation that does not rely of the assumption that the needle is the brightest linear object in the image.

This object is solved by the classification-based medical image segmentation apparatus of claim 1, and by the computer-readable medium embodying a computer program for classification-based identification of a medical image of claim 16. Advantageous embodiments are defined by the dependent claims.

In sub-aspects or related aspects, US beam steering is employed to enhance the appearance of specular reflectors in the image. Next, a pixel-wise needle classifier trained from previously acquired ground truth data is applied to segment the needle from the tissue background. Finally, a Radon or Hough transform is used to detect the needle pose. The segmenting is accomplished via statistical boosting of wavelet features. The whole process of acquiring an image, segmenting the needle, and displaying an image with a visually enhanced and artifact-free needle-only overlay is done automatically and without the need for user intervention.

Validation results using ex-vivo and clinical datasets show enhanced detection in challenging ex-vivo and clinical datasets where sub-optimal needle position and tissue artifacts cause intensity based segmentation to fail.

Details of the novel, real time classification-based medical image segmentation are set forth further below, with the aid of the following drawings, which are not drawn to scale.

### BRIEF DESCRIPTION OF THE DRAWINGS

FIG. 1 is a schematic and conceptual diagram of an exemplary real time classification-based medical image segmentation apparatus, in accordance with the present invention;
FIG. 2 is a conceptual diagram exemplary of the training, and clinical performance, of a statistical boosted classifier, and its use, in accordance with the present invention;
FIG. 3 is a conceptual diagram of a type of needle localization in accordance with the present invention;
FIG. 4 is a pair of flow charts of subroutines usable in a version of the present invention; and
FIG. 5 is a flow chart of a main routine demonstrating a clinical operation in accordance with the present invention.

### DETAILED DESCRIPTION OF EMBODIMENTS

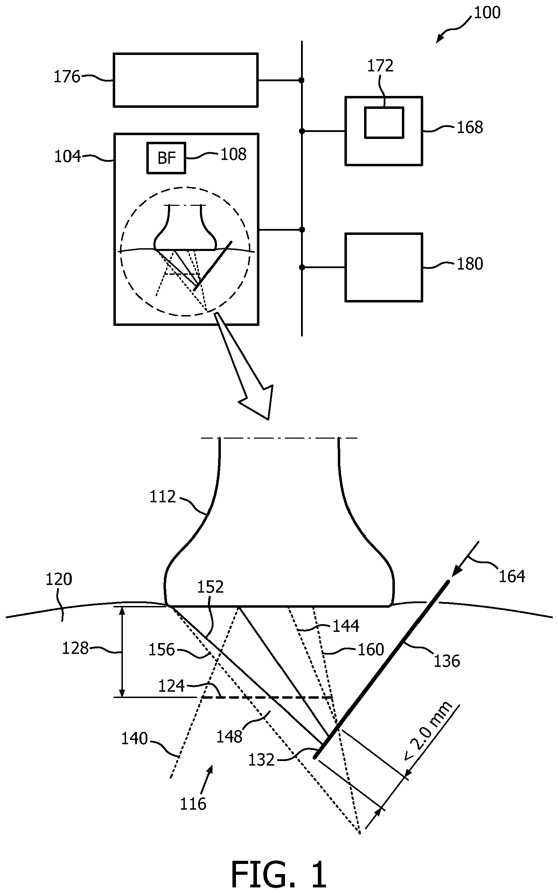

FIG. 1 depicts, by way of illustrative and non-limitative example, a real time classification-based medical image segmentation apparatus 100. It includes an ultrasound image acquisition device 104, such as a scanner. The device 104 includes a beamformer 108 and an ultrasound imaging probe 112. The probe 112 may be a linear array probe. It can be set with a field of view 116, in body tissue 120, that is defined by a lateral span 124 at any given imaging depth 128. The apparatus 110 can use the probe 112 to detect, in real time, entry of at least a portion 132 of a medical needle 136 into the field of view 116. The field of view 116 is defined by two boundary lines 140, 144. Detection of the needle 136 can occur with as little as 2.0 millimeters of the needle 136 being inserted into the field of view 116. This allows for earlier detection of the needle than available from existing methodologies. To improve the image of the needle 136, the current field of view 116 may change to a new field of view 148 for steering an ultrasound beam 152 into incidence upon the needle 136 at an angle of 90 degrees. The steered field of view 148 is shown in FIG. 1 with two boundary lines 156, 160. Beam steering to achieve normality with the needle 136 does not always require a change in the field of view 116. The improved image of the needle can be transferred to the overall image in the original field of view 116. This is done because the steering to achieve normality with the needle 136 slightly diminishes imaging quality in the resulting image overall, although enhancing visualization of the needle in particular. The apparatus 100 is designed for use in at least one of medical treatment and medical diagnosis. The needle 136 may, for example, be used to deliver medicament injected in an intra-body direction 164, as shown by the arrow. Biopsy, nerve block, and fluid aspiration are examples of other procedures where needle pose, position and movement are likewise monitored in real time.

The apparatus 100 further includes machine-learning-based-classification circuitry 168 that embodies a boosted classifier 172, such as Adaboost™ which is the most well-known statistical boosting algorithm.

For user interaction in monitoring via live imaging, the apparatus also includes a display 176 and user controls 180.

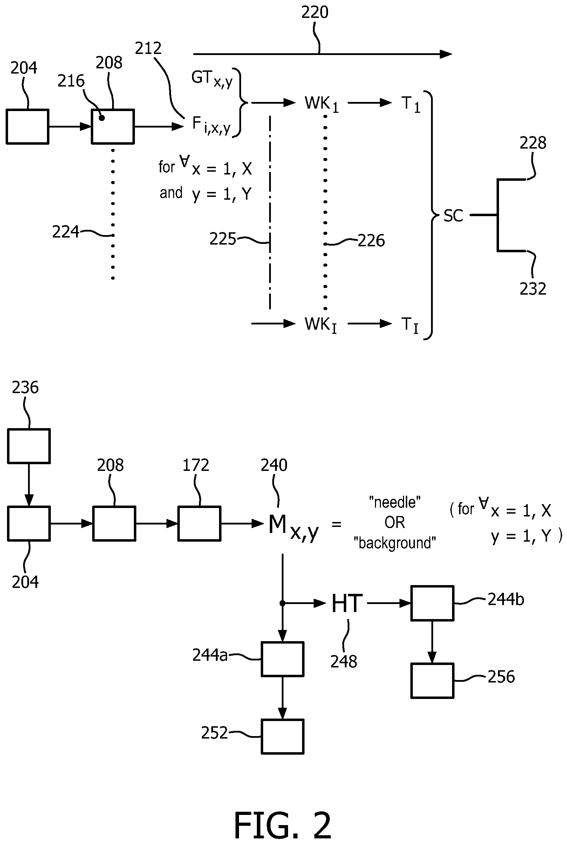

FIG. 2 conceptually portrays an exemplary version of training, and clinical performance, of the boosted classifier 172, and its use. To train the classifier 172, two-dimensional Log-Gabor wavelets (or "filters") 204 are applied to a needle image 208. The needle image 208 has been acquired via beam steering, as discussed in more detail further below, and by utilizing ultrasound frequencies lower than those typically used in B-mode imaging. The output from applying the wavelets 204 is a set of wavelet feature parameters F_{i,x,y} 212 for respective wavelet features Fᵢ and respective pixels (x,y) 216 of the needle image 208. The ground truth GT_{x,y} for each pixel 216, on whether it is part of the needle or part of the background is, or has been, determined. F_{1,x,y} and GT_{x,y} are part of a "weak classifier", WK₁. Multiple weak classifiers are combined, i.e., boosted 220, to provide a strong, or "boosted", classifier. An alternative to this technique would be to use pixel intensity to decide whether the pixel 216 is needle or background. However, because of artifacts in the needle image 208, the intensity based thresholding in not robust enough to effectively classify the needle 136. The above steps in training the boosted classifier 172 are repeated for each of the needle images 208 in the training dataset, as represented in FIG. 2 by the broken downward line 224. The above-mentioned weak classifier WK₁ is built up by using the feature parameters F_{1,x,y} each labeled with its respective ground truth GT_{x,y}. In particular, for a parameter from among F_{1,x,y}, the optimal threshold T₁ is found that delivers, in view of GT_{x,y}, the minimum classification error. The parameters to be thresholded essentially incorporate information about the shape of needle, angle of needle, texture and also the intensity. In addition, the training phase also provides information about what the needle doesn't look like, i.e., what are the characteristics of the background image and muscle texture. All of the above processing is repeated for each feature F₂ through F_{I}, as represented in FIG. 2 by the downward dot-dashed line 225. This is done to yield weak classifiers WK₂ through WK_{I}, as represented in FIG. 2 by the downward dotted line 226, and to correspondingly yield optimal thresholds T₂ through T_{I}. The weak classifiers WKᵢ are combined through appropriate weighting in forming a strong classifier SC which, during the clinical procedure, yields a binary output - "needle" 228 or "background" 232 for the pixel (x,y) 216. In effect, a set of weak hypotheses are combined into a strong hypothesis.

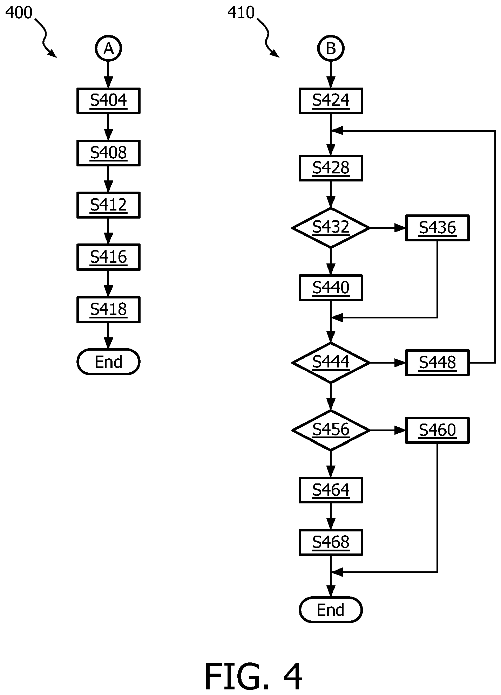

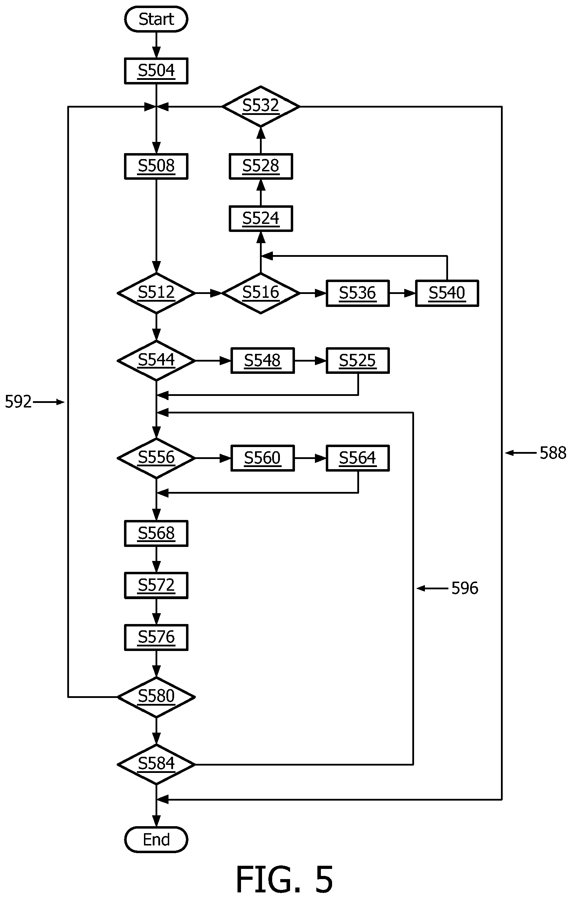

In the clinical procedure, the wavelets 204 are oriented incrementally in different angular directions as part of a sweep through an angular range, since 2D Log-Gabor filters can be oriented to respond to the spatial frequencies in different directions. At each increment, a respective needle image 208 is acquired at the current beam angle 236; the oriented wavelet 204 is applied, thereby operating on the image, to derive information, i.e., F_{i,x,y} 212, from the image; and the above-described segmentation operates on the derived information. In the latter step, the boosted classifier 172 outputs a binary pixel-map 240 M_{x,y} whose entries are apportioned between needle pixels and background pixels. Depending on the extraction mode chosen by the operator, or depending on the implementation, the needle portion of the map 240 can be extracted 244a and directly overlaid 252 onto a B-mode image, or a line detection algorithm such a Radon transform or Hough transform (HT) 248 can be used to derive a position and angle of the needle 136. In this latter case, a fresh needle image can be acquired, the background then being masked out, and the resulting, extracted 244b "needle-only" image superimposed 256 onto a current B-mode image. Thus, the extraction mode can be set for "pixel map" or "ultrasound image." It is reflected in steps S432, S456 and S556 which are discussed further below in connections with FIGs. 4 and 5. Although a Log-Gabor filter is described above, other imaging filters such as the Gabor filter can be used instead.

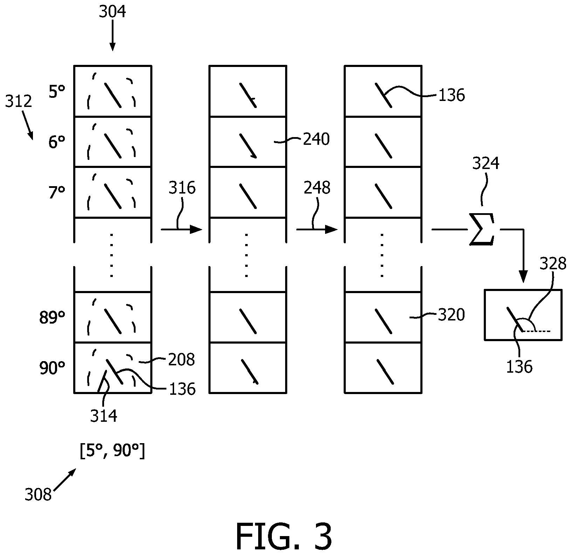

FIG. 3 further illustrates exemplary details on the clinical procedure. The above-described incremental sweep 304 is, in the displayed example, through a range 308 of angles. Each increment 312 is shown, in FIG. 3, next to the needle image 208 acquired at the corresponding beam angle. Any elongated, linear, and specular objects 314 in the image 208 are distinguished from the needle 136, due to the robustness of the segmentation proposed herein. A segmentation 316 is run, by the boosted classifier 172, on each needle image 208, generating respective pixel maps 240. The Hough transform 248 is applied to each pixel map 240. The resulting line outputs 320 are summed 324, as in summing the angle/offset bins. This determines an estimate of the offset, corresponding to the position, of the needle 136. It also determines the angle 328 of the needle 136. As mentioned herein above, the needle position and angle can be used in forming a displayable B-mode image with a "needle-only" overlay or, alternatively, the needle parts of the pixel maps 240 can be used provide needle pixels as the overlay.

Subroutines callable for performing the clinical procedure are shown in exemplary implementations in FIG. 4.

In a first subroutine 400, the wavelets 204 are oriented for the current beam angle 236 (step S404). The needle image 208 is acquired (step S408). The wavelets 204 are applied to the needle image 208 (step S412). The output is processed by the boosted statistical classifier 172 (step S416). The binary pixel map 240 is formed for the needle image 208 (step S418).

In a second subroutine 410, the beam angle 236 is initialized to 5° (step S424). The first subroutine 400 is invoked (step S428), to commence at entry point "A" in FIG. 4. If the pixel map 240 is being directly overlaid (step S432), the needle pixels and pixel-specific confidence values, for the needle pixels, are stored (step S436). Each of the weak classifiers WKᵢ returns, based on whether the threshold Tᵢ is met, either -1, representing background, or +1, representing needle. The strong classifier SC calculates a weighted sum of these values. If the sum is positive, the pixel 216 is deemed to be part of the needle 136. Otherwise, the pixel 216 is deemed to be part of the background. However, the sum also is indicative of a confidence in the strong hypothesis. The closer the sum is to +1, the more confidence the decision of "needle" is accorded. The needle pixels, along with their corresponding confidence values, are recorded at this time for later generating a robust pixel map based on confidence values. Alternatively, the pixel map 240 might not be directly overlaid (step S432). In one embodiment, for example, direct overlaying of the pixel map 240 maybe intended as a display option alternative to a main process of overlaying a needle-only ultrasound image. If, accordingly, the pixel map 240 is not being directly overlaid (step S432), the output of the Hough transform 248 at the current beam angle 236 is added to that at the previous beam angle, if any, to create a running sum of the transform output (step S440). In either event, i.e., pixel map overlay or not, if the current beam angle 236 is less than 90° (step S444), the current beam angle is incremented (step S448) and return is made to the segmenting step S428 (step S452). Otherwise, if the current beam angle 236 is 90° (step S444), processing again depends on whether the pixel map 240 is being directly overlaid (step S456). If the pixel map 240 is being directly overlaid (step S456), an optimal needle map is derived (step S460). In particular, the confidence values stored iteratively in step S436 are combined. For example, each negative confidence value can be made zero. The confidence maps generated for the respective beam angles 236 are added to create a summed map. The confidence values are then normalized to a range of pixel brightness values. If, on the other hand, the pixel map 240 is not being directly overlaid (step S456), the Hough transform summed output 324 from step S440 gives the needle offset (step S464). It also gives the angle 328 of the needle 136 (step S468).

FIG. 5 is, in the current example, the main routine of the clinical procedure. A needle-presence flag is initialized, i.e., cleared (step S504). The second subroutine 410 is called (step S508). It is determined whether or not a needle is present in the current field of view 116 (step S512). For instance, in the case of displaying the pixel map 240, the number of needle pixels and optionally their confidence levels may be subject to thresholding to determine whether the needle 136 is present. In the case of displaying an ultrasound overlay, the line bin totals of the summed Hough transform 324 may be thresholded to determine if the needle 136 is present. If it is determined that the needle 136 is not present (step S512), and if the needle-presence flag is not set (step S516), a B-mode image is acquired (step S524). It is displayed (step S528). If imaging is to continue (step S532), return is made to the segmenting step S508. If, on the other hand, the needle-presence flag is set (step S516), it is cleared (step S536). The user is notified that the needle 136 is no longer onscreen (step S540), and processing branches back to B-mode acquisition step S524. In the case that the needle is determined to be present (step S512), and the needle-presence flag is not set (step S544), the user is notified of the entry of the needle into the displayed image (step S548) and the needle-presence flag is set (step S552). At this point, whether or not the needle-presence flag was, or has just been, set (steps S544, S552), the processing path depends on whether the pixel map 240 is to used as an overlay (step S556). If the pixel map 240 is not to be used as an overlay (step S556), the needle angle 328 determined by the summed Hough transform 324 is used to steer the beam 152 to normality with the needle 136, thereby providing better visibility of the needle (step S560). Via the steered beam 152, a needle image 208 is acquired (step S564). Whether or not the pixel map 240 is to be used as an overlay, a B-mode image is acquired (step S568). A composite image is formed from the B-mode image and a superimposed needle-only image extracted from the needle image 208 or, in the case of a pixel map overlay, an extracted and superimposed set of the normalized confidence values from step S456 or other rendition of the pixel map 240 (step S572). The composite image is displayed (step S576). Periodically, i.e., iteratively after equally or unequally spaced apart periods of time, the needle 136 should be re-segmented as an update on its position and orientation. If the needle 136 is now to be re-segmented (step S580), processing returns to the segmenting step S508. Otherwise, if the needle 136 is not now to be re-segmented (step S580), but imaging is to continue (step S584), return is made to step S556.

The user notifications in steps S540 and S548 can be sensory, e.g., auditory, tactile or visual. For example, illuminations on a panel or display screen may, while in an "on" state, indicate that the respective mode of operation is active.

A needle-presence-detection mode 588 of operation corresponds, for example, to steps S512-S532.

A needle-insertion-detection mode 592 corresponds, for example, to steps S512 and S544-S552.

A needle visualization mode 596 corresponds, for example, to steps S556-S564. One can exit the needle visualization mode 596, yet remain in the needle-insertion-detection mode 592. If, at some time thereafter, the needle-insertion-detection mode 592 detects re-entry of the needle 136 into the field of view 116, the needle visualization mode 596 is re-activated automatically and without the need for user intervention. In the instant example, the needle-presence-detection mode 588 enables the needle-insertion-detection mode 592 and thus is always active during that mode 592.

The above modes 588, 592, 596 may be collectively or individually activated or deactivated by the user controls 180, and may each be incorporated into a larger overall mode.

Each of the above modes 588, 592, 596 may exist as an option of the apparatus 100, user-actuatable for example, or alternatively may be part of the apparatus without any option for switching off the mode.

It is the quality and reliability of the needle segmentation proposed herein above that enables the modes 588, 592, 596.

Although the proposed methodology can advantageously be applied in providing medical treatment to a human or animal subject, the scope of the present invention is not so limited. More broadly, techniques disclosed herein are directed to machine-learning-based image segmentation in vivo and ex vivo.

A classification-based medical image segmentation apparatus includes an ultrasound image acquisition device configured for acquiring, from ultrasound, an image depicting a medical instrument such as needle; and machine-learning-based-classification circuitry configured for using machine-learning-based-classification to, dynamically responsive to the acquiring, segment the instrument by operating on information derived from the image. The segmenting can be accomplished via statistical boosting of parameters of wavelet features. Each pixel of the image is identified as "needle" or "background." The whole process of acquiring an image, segmenting the needle, and displaying an image with a visually enhanced and artifact-free needle-only overlay may be performed automatically and without the need for user intervention. The reliable needle segmentation affords automatic setting of the optimal beam steering angle, time gain compensation, and the image processing parameters, resulting in enhanced visualization and clinical workflow.

While the invention has been illustrated and described in detail in the drawings and foregoing description, such illustration and description are to be considered illustrative or exemplary and not restrictive; the invention is not limited to the disclosed embodiments.

For example, the needle-insertion-detection mode 592 is capable of detecting at least part of the needle 136 when as little as 7 millimeters of the needle has been inserted into the body tissue, and, as mentioned herein above, 2.0 mm in the ultrasound field of view.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Any reference signs in the claims should not be construed as limiting the scope.

A computer program can be stored momentarily, temporarily or for a longer period of time on a suitable computer-readable medium, such as an optical storage medium or a solid-state medium. Such a medium is non-transitory only in the sense of not being a transitory, propagating signal, but includes other forms of computer-readable media such as register memory, processor cache and RAM

A single processor or other unit may fulfill the functions of several items recited in the claims. The mere fact that certain measures are recited in mutually different dependent claims does not indicate that a combination of these measures cannot be used to advantage.

## Claims

1. A classification-based medical-image identification apparatus (100) comprising:
an ultrasound image acquisition device (104) configured for acquiring, by means of ultrasound, an image depicting a medical instrument; and
a classification circuitry (168) configured to, dynamically responsive to said acquiring, segment the medical instrument by operating on information derived from said image,
**characterized in that**
the classification circuitry is machine-learning-based-classification circuitry (168), which comprises a boosted statistical classifier (172) configured for using machine-learning-based-classification to segment the medical instrument, and
**in that** the apparatus is configured for
- via the ultrasound image acquisition device, dynamically performing, automatically, without need for user intervention, said acquiring incrementally, in a sweep, over a range (308) of ultrasound beam angles (236) for corresponding depictions of the medical instrument, the segmenting of said depictions being dynamically responsive to the incremental acquiring,
- applying, increment (312) by increment, a line detection algorithm to output of said segmenting for respective ones of said depictions, and
- adding outputs of said algorithm to derive an orientation of the medical instrument.

2. The apparatus of claim 1, wherein said using comprises performing statistical boosting of parameters (212) of wavelet features.

3. The apparatus of claim 1, further configured for performing said segmenting (316) of said depictions depiction-by-depiction.

4. The apparatus of claim 1, wherein said segmenting of said depictions uses, in correspondence with said ultrasound beam angles, different orientations of an imaging filter (step S404, S428, S448).

5. The apparatus of claim 1, further comprising an ultrasound imaging probe (112), said apparatus being configured for dynamically determining, based on an output of the segmenting, an orientation of the medical instrument with respect to said probe.

6. The apparatus of claim 1, wherein the medical instrument is a medical needle (136).

7. The apparatus of claim 6, wherein the orientation of the medical needle (136) comprises a needle offset and a needle angle (328).

8. The apparatus of claim 7, further configured for using the determined needle angle (328) to steer an ultrasound beam (152) to normality with the medical needle (136).

9. The apparatus of claim 8, further configured for:
- acquring (S564), via the steered ultrasound beam (152) a needle image (208);
- acquring (S568) a B-mode image; and
- forming (S572) a composite image from the B-mode image and a superimposed needle-only image extracted from the needle image (208).

10. The apparatus of claim 6, wherein said apparatus is designed for use in at least one of medical treatment and medical diagnosis.

11. The apparatus of claim 10, wherein said circuitry is configured for the segmenting in the circumstance that body tissue, in said image, that surrounds the medical needle includes an anatomical structure (314) that is elongated, linear, and specular.

12. The apparatus of claim 11, configured for said segmenting in said circumstance automatically, without need for user intervention (S508, S532).

13. The apparatus of claim 6, further comprising a display, said device comprising an ultrasound imaging probe having a field of view for spatially defining a span (124) of dynamic visualizing of body tissue via said display, said apparatus being further configured with a needle-presence-detection mode of operation, said apparatus being further configured for, while in said mode, automatically, without need for user intervention, deciding, based on output of the segmenting, that no needle is even partially present in said field of view.

14. The apparatus of claim 13, further configured for, responsive to said deciding that even the partial presence does not exist, user notification (S540), while in said mode, of the decision, the notifying being performed automatically, without need for user intervention.

15. The apparatus of claim 13, further configured for, while in said mode (596), detecting, based on said output, entry of at least part of a needle into said field of view and for, dynamically responsive to said detecting of said entry, commencing needle visualization mode which entails performance, by said apparatus, of beam steering for better visibility of the medical needle, and enhancing visualization, on said display, of the medical needle.

16. A computer-readable medium embodying a computer program for classification-based identification of a medical image, said program having instructions executable by a processor for performing a plurality of acts, among said plurality there being the acts of:
dynamically performing, automatically, without need for user intervention, an incremental acquisition, in a sweep, over a range (308) of angles (236), from ultrasound (152), of corresponding images depicting a medical instrument;
using machine-learning-based statistical classification to, dynamically responsive to said incremental acquiring, segment said depictions of the medical instrument by operating on information derived from said corresponding images depicting a medical instrument;
applying, increment (312) by increment, a line detection algorithm to output of said segmenting for respective ones of said depictions; and
adding outputs of said algorithm to derive an orientation of the medical instrument.

## Patentansprüche

1. Klassifizierungsbasierte medizinische Bildidentifizierungseinrichtung (100), umfassend:
eine Ultraschallbildbeschaffungsvorrichtung (104), die konfiguriert ist, mittels Ultraschall ein Bild zu beschaffen, das ein medizinisches Instrument abbildet; und
einen Klassifizierungsschaltkreis (168), der konfiguriert ist, dynamisch in Reaktion auf das Beschaffen, das medizinische Instrument durch Bearbeiten von Informationen zu unterteilen, die vom Bild abgeleitet sind,
**dadurch gekennzeichnet, dass**
der Klassifizierungsschaltkreis ein maschinenlernbasierter Klassifizierungsschaltkreis (168) ist, der einen verstärkten statistischen Klassifizierer (172) umfasst, der konfiguriert ist, maschinenlernbasierte Klassifizierung zu verwenden, um das medizinische Instrument zu unterteilen, und
dadurch, dass die Einrichtung konfiguriert ist zum
- über die Ultraschallbildbeschaffungsvorrichtung, dynamisches automatisches Durchführen, ohne einen Benutzereingriff zu benötigen, des Beschaffens, schrittweise, auf einen Schwung, über einen Bereich (308) von Ultraschallstrahlwinkeln (236) für entsprechende Abbildungen des medizinischen Instruments, wobei das Unterteilen der Abbildungen dynamisch auf das schrittweise Beschaffen reagiert,
- Anwenden, Schritt (312) für Schritt, eines Linienerfassungsalgorithmus, um das Unterteilen für jeweilige der Abbildungen auszugeben, und
- Hinzufügen von Ausgängen des Algorithmus, um eine Ausrichtung des medizinischen Instruments abzuleiten.

2. Einrichtung nach Anspruch 1, wobei das Verwenden ein Durchführen statistischer Verstärkung von Parametern (212) von Waveletmerkmalen umfasst.

3. Einrichtung nach Anspruch 1, weiter konfiguriert, das Unterteilen (316) der Abbildungen Abbildung für Abbildung durchzuführen.

4. Einrichtung nach Anspruch 1, wobei das Unterteilen der Abbildungen in Übereinstimmung mit den Ultraschallstrahlwinkeln verschiedene Ausrichtungen eines Abbildungsfilters verwendet (Schritt S404, S428, S448).

5. Einrichtung nach Anspruch 1, weiter umfassend eine Ultraschallabbildungssonde (112), wobei die Einrichtung zum dynamischen Bestimmen, basierend auf einem Ausgang der Unterteilung, eine Ausrichtung des medizinischen Instruments in Bezug auf die Sonde konfiguriert ist.

6. Einrichtung nach Anspruch 1, wobei das medizinische Instrument eine medizinische Nadel (136) ist.

7. Einrichtung nach Anspruch 6, wobei die Ausrichtung der medizinischen Nadel (136) einen Nadelversatz und einen Nadelwinkel (328) umfasst.

8. Einrichtung nach Anspruch 7, weiter konfiguriert, den bestimmten Nadelwinkel (328) zu verwenden, um einen Ultraschallstrahl (152) zu Normalität mit der medizinischen Nadel (136) zu lenken.

9. Einrichtung nach Anspruch 8, weiter konfiguriert zum:
- Beschaffen (S564), über den gelenkten Ultraschallstrahl (152), eines Nadelbilds (208);
- Beschaffen (S568) eines B-Modus-Bilds; und
- Bilden (S572) eines zusammengesetzten Bilds vom B-Modus-Bild und einem überlagerten Nur-Nadel-Bild, das vom Nadelbild (208) extrahiert ist.

10. Einrichtung nach Anspruch 6, wobei die Einrichtung zur Verwendung in zumindest einer von medizinischer Behandlung und medizinischer Diagnose gestaltet ist.

11. Einrichtung nach Anspruch 10, wobei der Schaltkreis für die Unterteilung unter dem Umstand konfiguriert ist, dass Körpergewebe, im Bild, das die medizinische Nadel umgibt, eine anatomische Struktur (314) beinhaltet, die länglich, linear und spiegelnd ist.

12. Einrichtung nach Anspruch 11, die unter dem Umstand automatisch für die Unterteilung konfiguriert ist, ohne einen Benutzereingriff zu benötigen (S508, S532).

13. Einrichtung nach Anspruch 6, weiter umfassend eine Anzeige, wobei die Vorrichtung eine Ultraschallabbildungssonde mit einem Blickfeld umfasst, um räumlich eine Spanne (124) von dynamischem Visualisieren von Körpergewebe über die Anzeige zu definieren, wobei die Einrichtung weiter mit einem Nadelgegenwartserfassungsbetriebsmodus konfiguriert ist, wobei die Einrichtung weiter konfiguriert ist, während in dem Modus, automatisch, ohne einen Benutzereingriff zu benötigen, basierend auf Ausgang der Unterteilung, zu entscheiden, dass keine Nadel auch nur teilweise im Blickfeld gegenwärtig ist.

14. Einrichtung nach Anspruch 13, weiter konfiguriert, in Reaktion auf das Entscheiden, dass nicht einmal die teilweise Gegenwart besteht, zur Benutzerbenachrichtigung (S540), während in dem Modus, über die Entscheidung, wobei das Benachrichtigen automatisch, ohne einen Benutzereingriff zu benötigen, durchgeführt wird.

15. Einrichtung nach Anspruch 13, weiter konfiguriert zum, während in dem Modus (596), Erfassen, basierend auf dem Ausgang, eines Eintritts zumindest eines Teils einer Nadel in das Blickfeld und zum, dynamisch in Reaktion auf das Erfassen des Eintritts, Einleiten eines Nadelvisualisierungsmodus, der eine Durchführung, durch die Einrichtung, von Strahllenkung für bessere Sichtbarkeit der medizinischen Nadel mit sich bringt und Visualisierung, auf der Anzeige, der medizinischen Nadel verbessert.

16. Computerlesbares Medium, das ein Computerprogramm für klassifizierungsbasierte Identifizierung eines medizinischen Bilds verkörpert, wobei das Programm Anweisungen hat, die von einem Prozessor zum Ausführen einer Vielzahl von Handlungen ausführbar sind, wobei unter der Vielzahl die Handlungen sind vom:
dynamischen automatischen Durchführen, ohne einen Benutzereingriff zu benötigen, einer schrittweisen Beschaffung, in einem Schwung, über einen Bereich (308) von Winkeln (236), von Ultraschall (152), entsprechender Bilder ,die ein medizinisches Instrument abbilden;
Verwenden von maschinenlernbasierter statistischer Klassifizierung, um, dynamisch in Reaktion auf das schrittweise Beschaffen, die Abbildungen des medizinischen Instruments durch Bearbeiten von Informationen, die von den entsprechenden Bildern abgeleitet sind, die ein medizinisches Instrument abbilden, zu unterteilen;
Anwenden, Schritt (312) für Schritt, eines Linienerfassungsalgorithmus, um das Unterteilen jeweiliger der Abbildungen auszugeben; und
Hinzufügen von Ausgängen des Algorithmus, um eine Ausrichtung des medizinischen Instruments abzuleiten.

## Revendications

1. Appareil d'identification d'images médicales se basant sur la classification (100) comprenant :
un dispositif d'acquisition d'images par ultrasons (104) configuré pour acquérir, au moyen d'ultrasons, une image décrivant un instrument médical ; et
un circuit de classification (168) configuré pour, en répondant de manière dynamique à ladite acquisition, segmenter l'instrument médical en opérant sur des informations déduites de ladite image,
**caractérisé en ce que**
le circuit de classification est un circuit de classification se basant sur l'apprentissage machine (168), qui comprend un classeur statistique renforcé (172) configuré pour utiliser une classification se basant sur l'apprentissage machine pour segmenter l'instrument médical, et
**en ce que** l'appareil est configuré pour
- via le dispositif d'acquisition d'images par ultrasons, mener à bien de manière dynamique, automatiquement, sans nécessiter d'intervention de l'utilisateur, ladite acquisition de manière incrémentielle, lors d'un balayage, sur une plage (308) d'angles de faisceaux d'ultrasons (236) pour mettre en correspondance des descriptions de l'instrument médical, la segmentation desdites descriptions répondant de manière dynamique à l'acquisition incrémentielle,
- appliquer, incrément (312) par incrément, un algorithme de détection de lignes pour la production de ladite segmentation pour les descriptions respectives desdites descriptions, et
ajouter des productions dudit algorithme pour déduire une orientation de l'instrument médical.

2. Appareil selon la revendication 1, dans lequel ladite utilisation comprend le fait de mener à bien un renforcement statistique de paramètres (212) de caractéristiques d'ondelettes.

3. Appareil selon la revendication 1, configuré en outre pour mener à bien ladite segmentation (316) desdites descriptions description par description.

4. Appareil selon la revendication 1, dans lequel ladite segmentation desdites descriptions utilise, en correspondance avec lesdits angles de faisceaux d'ultrasons, différentes orientations d'un filtre d'imagerie (étape S404, S428, S448).

5. Appareil selon la revendication 1, comprenant en outre une sonde d'imagerie à ultrasons (112), ledit appareil étant configuré pour déterminer de manière dynamique, sur la base d'une production de la segmentation, une orientation de l'instrument médical par rapport à ladite sonde.

6. Appareil selon la revendication 1, dans lequel l'instrument médical est une aiguille médicale (136).

7. Appareil selon la revendication 6, dans lequel l'orientation de l'aiguille médicale (136) comprend un décalage d'aiguille et un angle d'aiguille (328).

8. Appareil selon la revendication 7, configuré en outre pour utiliser l'angle d'aiguille (328) déterminé pour piloter un faisceau d'ultrasons (152) jusqu'à la perpendicularité avec l'aiguille médicale (136).

9. Appareil selon la revendication 8, configuré en outre pour :
- acquérir (S564), via le faisceau d'ultrasons (152) piloté une image d'aiguille (208) ;
- acquérir (S568) une image en mode B ; et
- former (S572) une image composite à partir de l'image en mode B et d'une image uniquement d'aiguille superposée extraite de l'image d'aiguille (208).

10. Appareil selon la revendication 6, dans lequel ledit appareil est conçu pour être utilisé dans au moins l'un d'un traitement médical et d'un diagnostic médical.

11. Appareil selon la revendication 10, dans lequel ledit circuit est configuré pour la segmentation dans la circonstance où un tissu corporel, dans ladite image, qui entoure l'aiguille médicale inclut une structure anatomique (314) qui est allongée, linéaire et spéculaire.

12. Appareil selon la revendication 11, configuré pour ladite segmentation dans ladite circonstance automatiquement, sans nécessiter d'intervention de l'utilisateur (S508, S532).

13. Appareil selon la revendication 6, comprenant en outre un écran, ledit dispositif comprenant une sonde d'imagerie à ultrasons ayant un champ visuel pour définir dans l'espace une portée (124) de visualisation dynamique de tissu corporel via ledit écran, ledit appareil étant configuré en outre avec un mode opératoire de détection de présence d'aiguille, ledit appareil étant configuré en outre pour, lorsqu'il est dans ledit mode, décider automatiquement, sans nécessiter d'intervention de l'utilisateur, sur la base d'une production de la segmentation, qu'aucune aiguille n'est présente même partiellement dans ledit champ visuel.

14. Appareil selon la revendication 13, configuré en outre pour, en réponse à ladite décision que même la présence partielle n'existe pas, une notification d'utilisateur (S540), lorsqu'il est dans ledit mode, de la décision, la notification étant menée à bien automatiquement, sans nécessiter d'intervention de l'utilisateur.

15. Appareil selon la revendication 13, configuré en outre pour, lorsqu'il est dans ledit mode (596), détecter, sur la base de ladite production, l'entrée d'au moins une partie d'une aiguille dans ledit champ visuel et pour, en répondant de manière dynamique à ladite détection de ladite entrée, commencer un mode de visualisation d'aiguille qui entraîne une performance, par ledit appareil, de pilotage de faisceaux pour une meilleure visibilité de l'aiguille médicale, et améliorer la visualisation, sur ledit écran, de l'aiguille médicale.

16. Support lisible sur un ordinateur mettant en œuvre un programme informatique pour une identification se basant sur la classification d'une image médicale, ledit programme ayant des instructions exécutables par une unité de traitement pour mener à bien une pluralité d'actions, parmi ladite pluralité étant incluses les actions consistant à :
mener à bien de manière dynamique, automatiquement, sans nécessiter d'intervention de l'utilisateur, une acquisition incrémentielle, lors d'un balayage, sur une plage (308) d'angles (236), à partir d'ultrasons (152), d'images correspondantes décrivant un instrument médical ;
utiliser une classification statistique basée sur l'apprentissage machine pour, en répondant de manière dynamique à ladite acquisition incrémentielle, segmenter lesdites descriptions de l'instrument médical en opérant sur des informations déduites desdites images correspondantes décrivant un instrument médical ;
appliquer, incrément (312) par incrément, un algorithme de détection de lignes pour la production de ladite segmentation pour les descriptions respectives desdites descriptions, et
ajouter des productions dudit algorithme pour déduire une orientation de l'instrument médical.
